# EUROPEAN PATENT SPECIFICATION

(11) **EP 2 022 982 B1**
(45) Date of publication and mention of the grant of the patent: **21.12.2016**
(21) Application number: 07112942.3
(22) Date of filing: 23.07.2007
(51) Int. Cl.: F04B 1/02, A61M 5/142

(54) **Volumetric pump**
Volumetrische Pumpe
Pompe volumétrique

(43) Date of publication of application: 11.02.2009
(73) Proprietor: ACIST Medical Systems, Inc., Eden Prairie, MN 55344 (US)
(72) Inventor: Mohiuddin, Khader, Medina, MN 55340 (US)
(74) Representative: Howe, Steven

(56) References cited:
- EP-A- 0 624 379
- WO-A1-2006/056828
- CH-A- 146 646
- DE-A1- 4 322 560
- US-A- 3 128 782
- US-B1- 6 224 572

## Description

### TECHNICAL FIELD

The present application relates to a volumetric pump that may be used within a medical drug or fluid delivery pump (such as an infusion pump, an IV pump, an enteral pump, or a parenteral pump). The volumetric pump may also be used within a powered contrast injection system for medical applications.

### BACKGROUND

Piston pumps with fluid modules are already part of the prior art. US 2004/101426 discloses a device comprising a cylindrical piston chamber whose upper and lower ends' profile have a specific gradient, said piston chamber containing a rotatable and axially movable pump piston. The profile of the upper and lower end surfaces of the piston has been determined to run concomitantly in contact with the respective two end surfaces of the chamber as the piston rotates. This rotation causes the piston to move alternately upwards and downwards permitting one-way suction and one-way propulsion of a fluid respectively into and out of the pump chambers. The rotational movement of the piston acts as a valve opening and closing alternately the inlet and outlet ports. The drawback of such system results essentially from the difficulties encountered when assembling the piston with the cylindrical chamber.

GB 2060131, US 4,767,399 and US 4,850,980 disclose a pumping mechanism device whose suction and propulsion phases are achieved by means of a bidirectional linear movement of a piston inside a chamber. Unlike US 2004/101426, such pumping mechanism has a device acting as a valve on the inlet/outlet ports which is independent of the piston's movement. Accordingly, the movement of the valve as well as its synchronization with the piston's movement requires more parts thus increasing the cost of the pumping mechanism. CH 146646 discloses a suction and force pump including opposed cylinders, each including a piston, provided on either side of pairs of intakes and discharges. Each intake and discharge has a ball valve that is alternately sealed and opened with the movement of the respective piston.

WO2006/056828 A1 discloses a piston pump with a disc having a combined bidirectional linear and angular movement.

### SUMMARY

In accordance with the present invention, there is provided a medical fluid delivery system according to claim 1.

In one embodiment, the medical fluid delivery system comprises a powered contrast media injection system capable of delivering contrast media and/or diluent to a patient.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

Figure 1 is a perspective view of a volumetric pump, not in accordance with the invention, with a piston located in a hollow cylinder, with the rotor removed.
Figure 2 is a perspective view of a rotor comprising an eccentric shaft of Figure 1.
Figure 3 is a cross-sectional view showing the engagement of this eccentric shaft in a receptacle adjacent the top of the piston.
Figure 3a shows a detail of Figure 3.
Figure 4 is a perspective view of the volumetric pump of Figure 1 at the beginning of a revolution cycle of the rotor.
Figure 4a is an axially sectioned rear view of Figure 4.
Figure 4b is a cross-sectional view taken on the line A-A in Figure 4a.
Figure 5 is a perspective view of the volumetric pump after a 90 degree rotation of the rotor.
Figure 5a is an axially sectioned rear view of Figure 5 and Figure 5b is a cross-sectional view taken on the line A-A in Figure 5a.
Figure 6 is a perspective view the volumetric pump after a 180 degree rotation of the rotor.
Figure 6a is an axially sectioned rear view of Figure 6.
Figure 6b is a cross-sectional view taken on the line A-A in Figure 6a.
Figure 7 is a perspective view of the volumetric pump after a 270 degree rotation of the rotor.
Figure 7a is an axially sectioned rear view of Figure 7.
Figure 7b is a cross-sectional view taken on the line A-A in Figure 7a.
Figure 8 is a perspective view of the volumetric pump, not in accordance with the invention comprising a piston head.
Figure 8a is a perspective view of said piston head connected to the shaft of the rotor.
Figure 8b is a perspective view of the piston.
Figure 9 is a perspective top view of the volumetric pump, not in accordance with the invention showing the pump in transparency without the rotor.
Figure 9a is a perspective bottom view showing the outside of the volumetric pump without the rotor.
Figure 10 is a perspective view of one of the two cylindrical parts constituting the hollow cylindrical housing.
Figure 10a is a perspective view of another rotatable element fitted into the cylindrical part of Figure 10.
Figure 11 is a front view of this rotatable element.

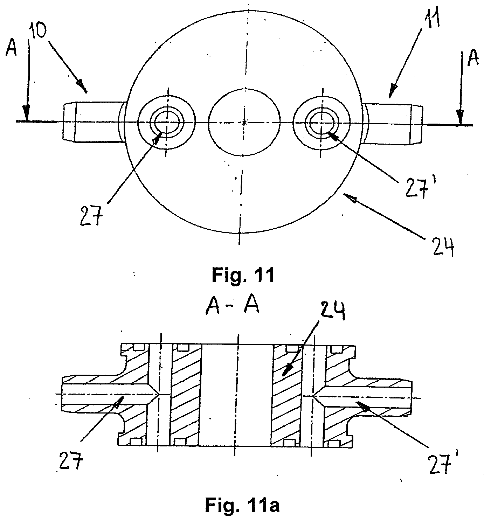

Figure 11 a a cross- sectional view of said element taken on the line A-A in Figure 11.

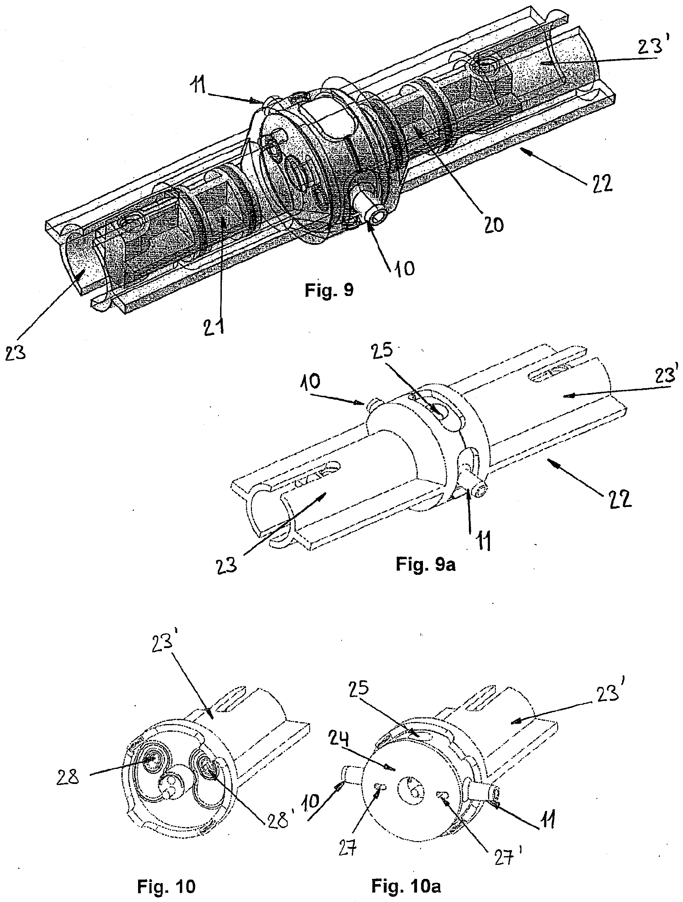

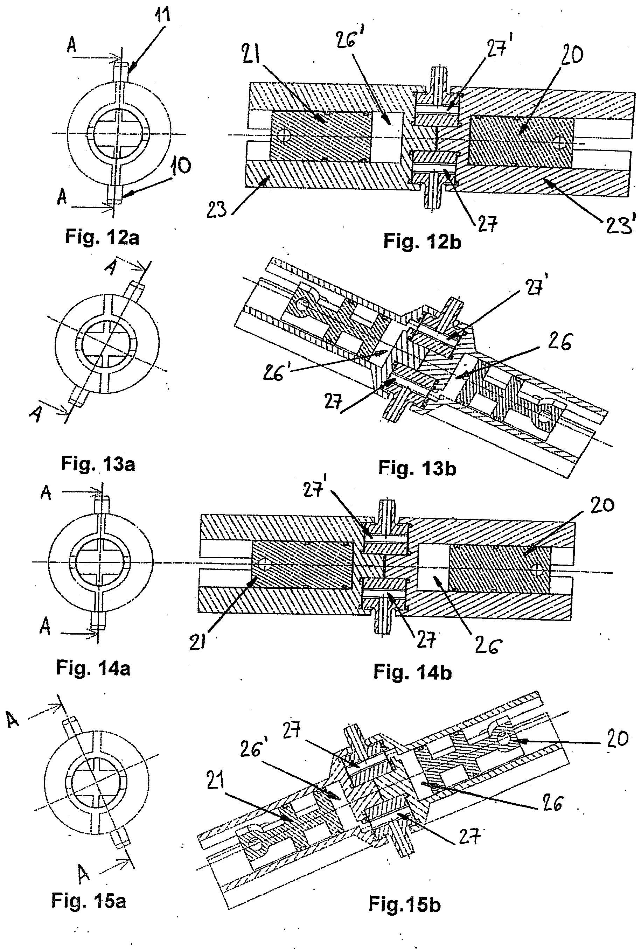

Figure 12a is an end view of Figure 9.
Figure 12b a cross-sectional view taken on the line A-A in Figure 12a at the beginning of a cycle.
Figure 13a is an end view of Figure 9.
Figure 13b a cross-sectional view taken on the line A-A in Figure 13a after a 90 degree rotation of the rotor.
Figure 14a is an end view of Figure 9.
Figure 14b a cross-sectional view taken on the line A-A in Figure 14a after 180 degree rotation of the rotor.
Figure 15a is an end view of Figure 9.
Figure 15b a cross-sectional view taken on the line A-A in Figure 15a after 270 degree rotation of the rotor.

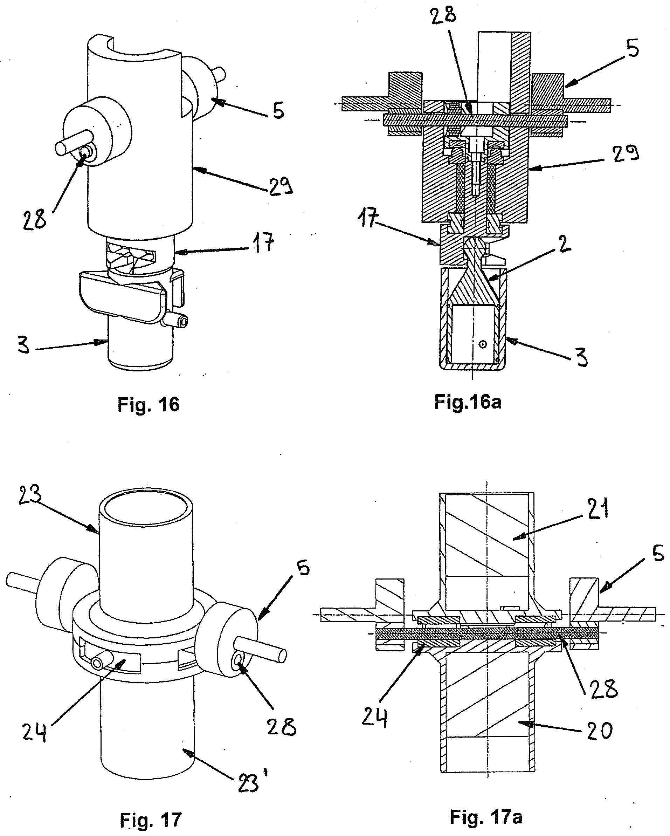

Figure 16 is a perspective view of the volumetric pump not in accordance with the invention.
Figure 16a is an axially sectioned view of Figure 16 taken along an axis connected to a least one rotor.
Figure 17 is a perspective view of the volumetric pump according to a further embodiment of the invention.
Figure 17a is an axially sectioned view of Figure 17 taken along an axis connected to at least one rotor.

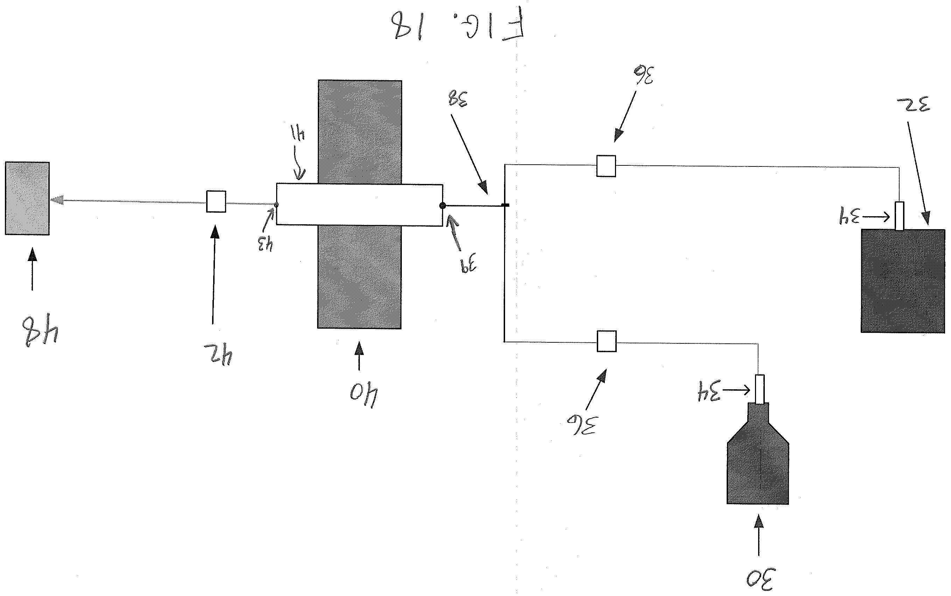

Figure 18 is a block-diagram view of a medical fluid delivery system that includes a volumetric pump.

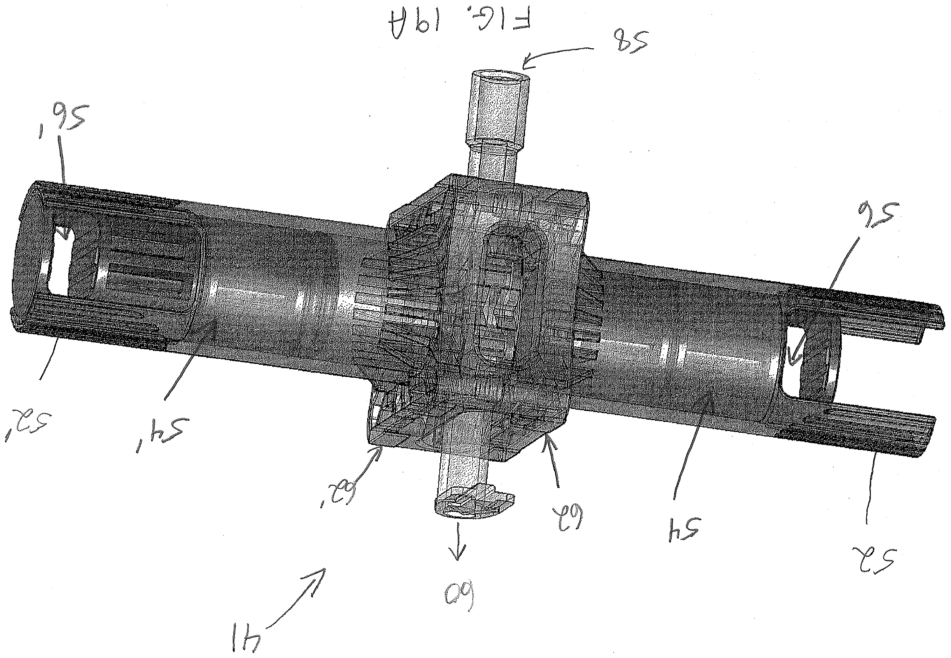

Figure 19a is a perspective view of a volumetric pump that may be used within the system shown in Figure 18.

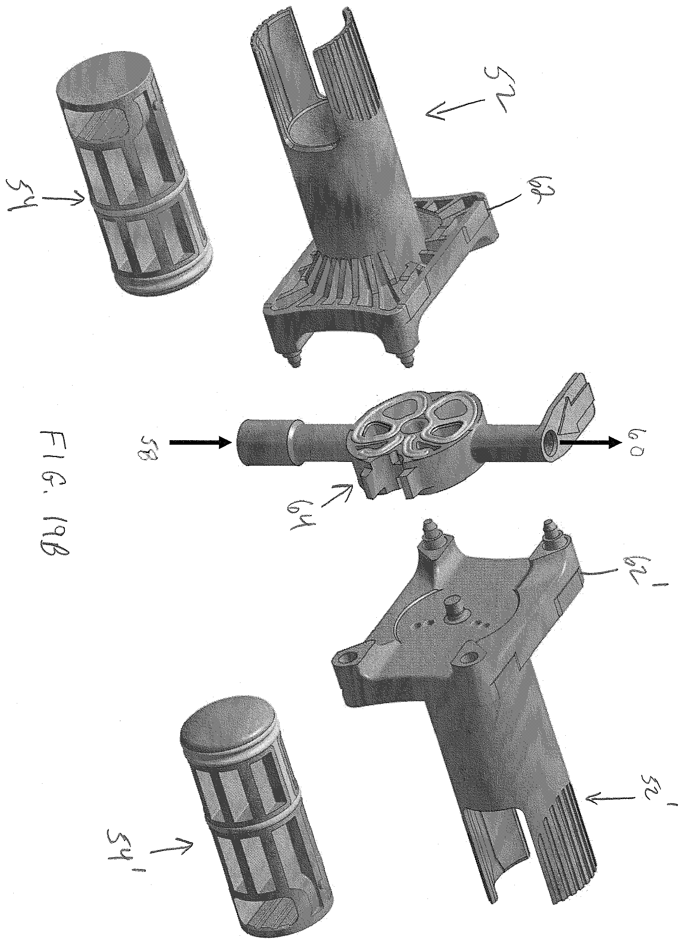

Figure 19b is a perspective view of individual components of the pump shown in Figure 19a.

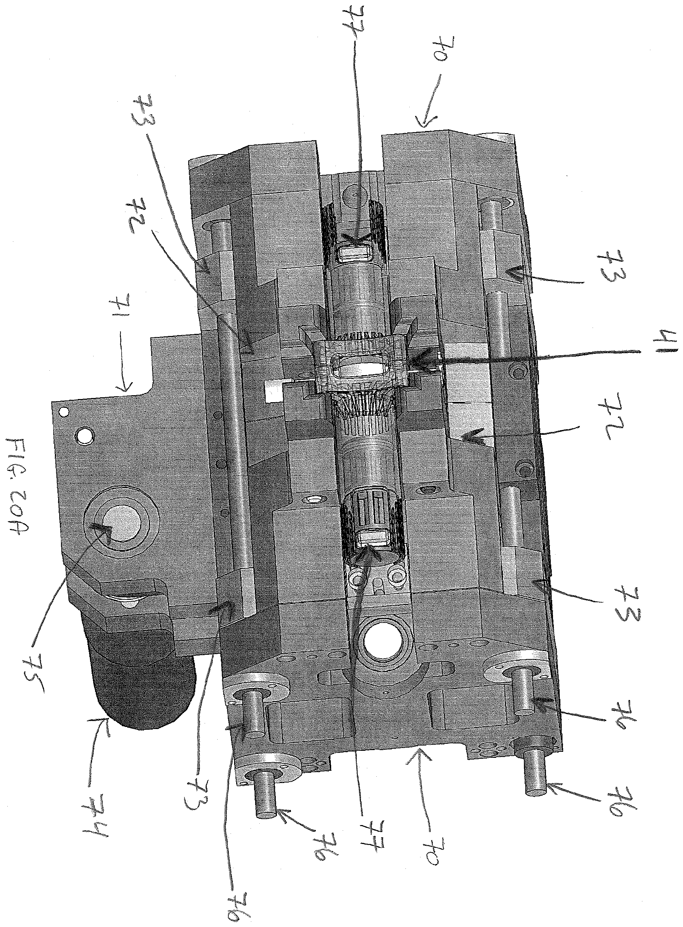

Figure 20a is a perspective view of a housing assembly that contains the volumetric pump of Figure 19a, according to one embodiment.

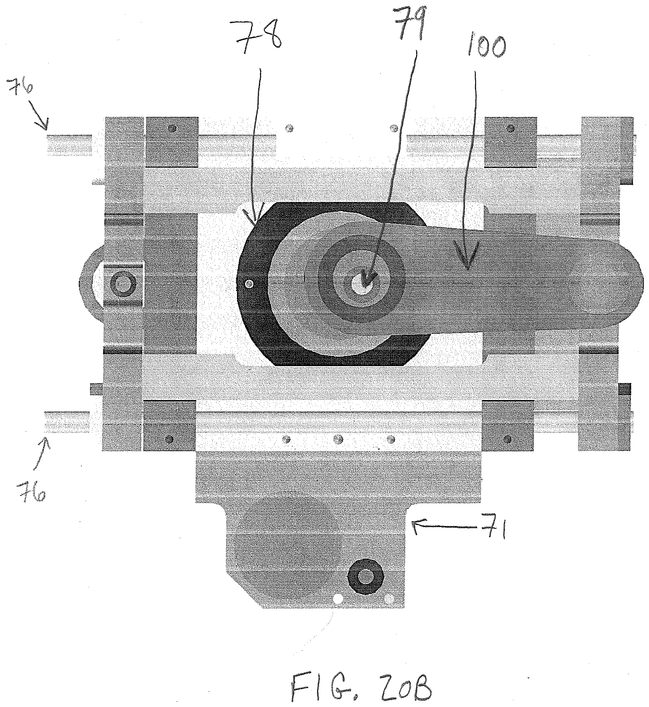

Figure 20b is view of a portion of the assembly shown in Figure 20a, according to one embodiment.

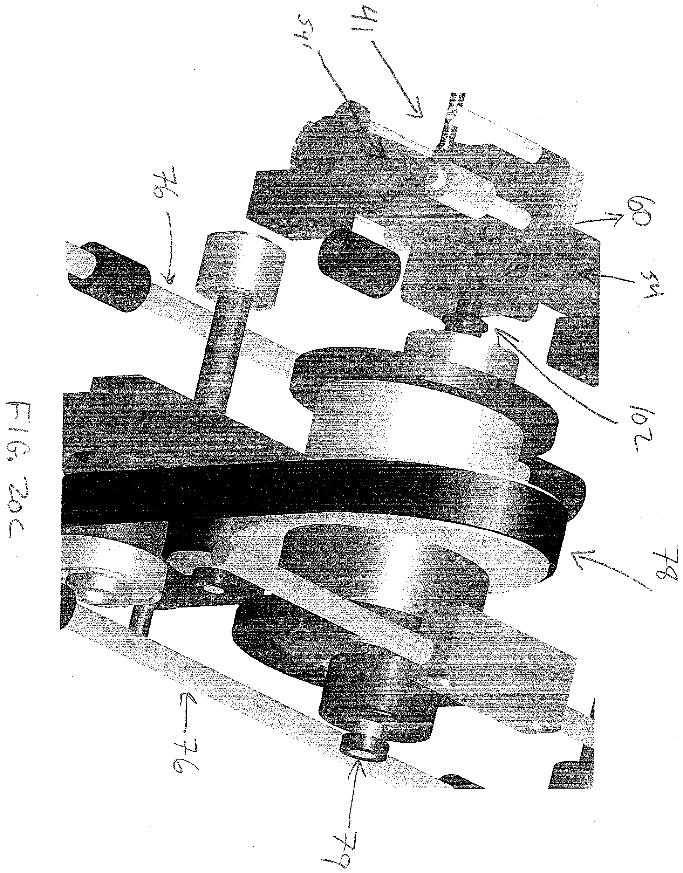

Figure 20c is a perspective view of a portion of the assembly shown in Figure 20a, according to one embodiment.

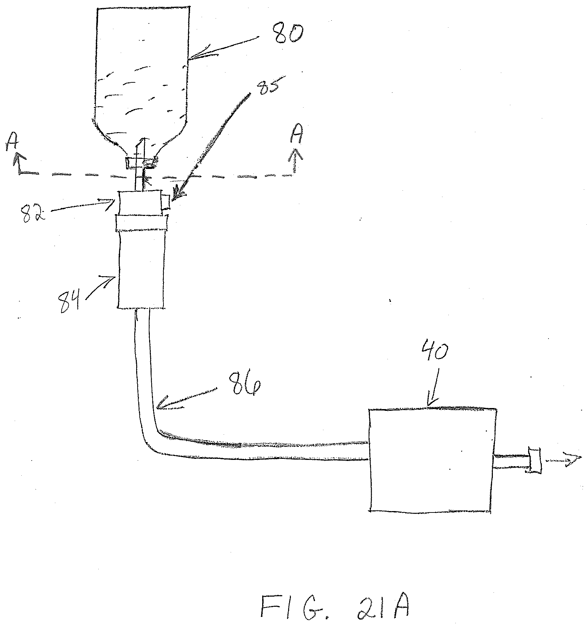

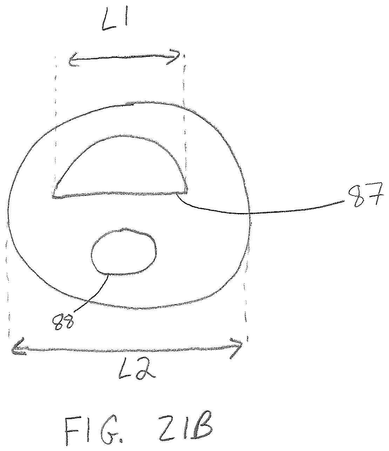

Figure 21 a is a view of a portion of the system shown in Figure 18, according to one embodiment.
Figure 21b is a cross-sectional view of a portion of the spike assembly that is shown in Figure 21a, according to one embodiment.

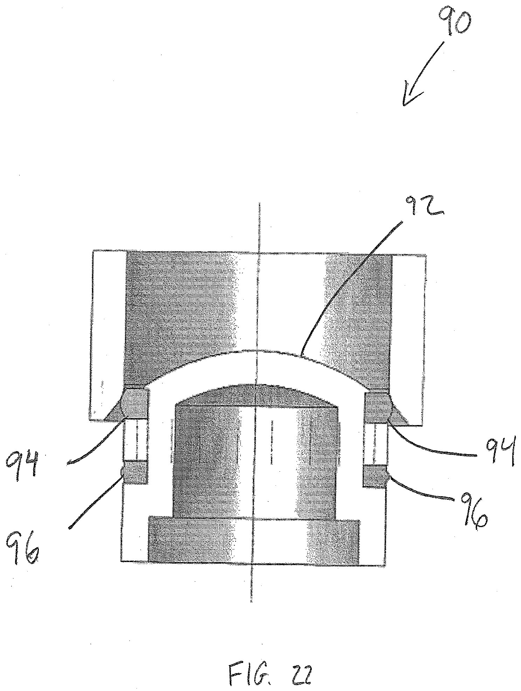

Figure 22 is a side sectional view of a portion of a piston that is included within the pump shown in Figure 19a, according to one embodiment.

### DETAILED DESCRIPTION

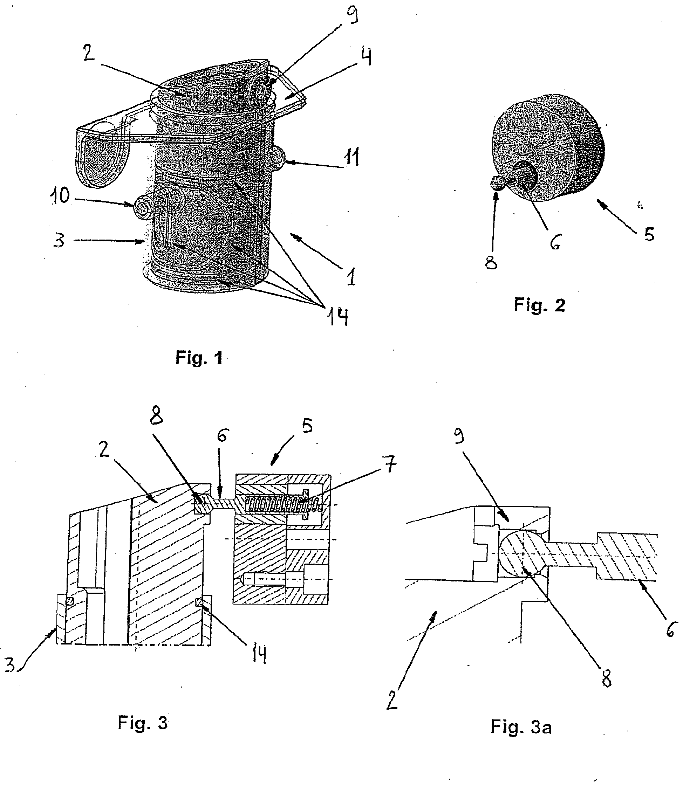

Figure 1 shows a volumetric pump 1 (not falling within the scope of the present invention) comprising a cylindrical piston 2 and a hollow cylinder 3 mounted on a support 4. This cylinder 3 has an upper open end wherein the piston 2 slidably fits. Piston 2 is actuated by a rotor 5 bearing an eccentric shaft 6 that is mounted on a spring 7.

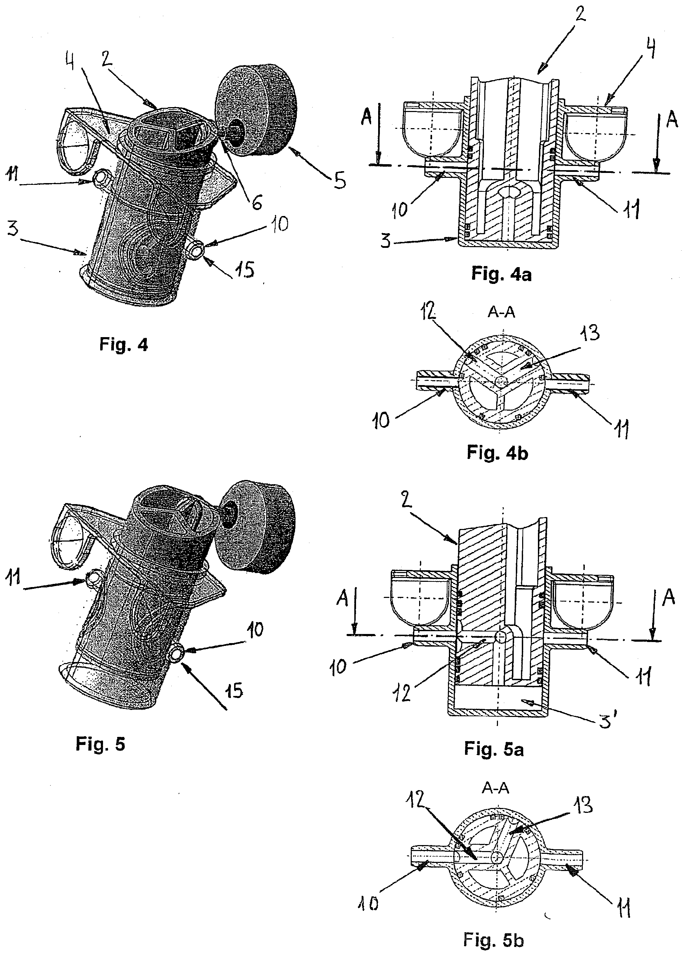

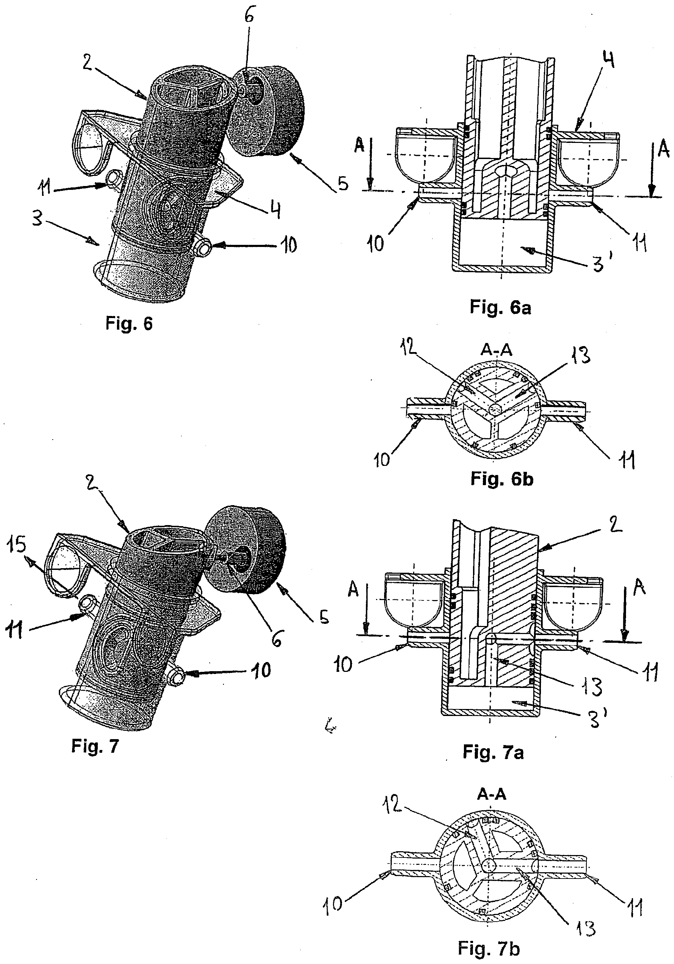

As shown by Figure 3 and Figure 3a, the shaft 6 ends with a spherical extremity 8 which is clipped into a piston receptacle 9 in order to transform the angular motion of the rotor 5 into a bi-directional linear and angular movement of the piston 2. This piston 2 slides to and fro inside the cylinder 3 while having a bi-directional angular movement. Shaft 6 transmits the movement of the piston 2 inside cylinder 3 as described below, while the spring 7 ensures a smooth articulation of the extremity 8 inside the receptacle 9. Spring 7 is compressed when the piston 2 reaches the ends of the suction and propulsion strokes Figure 4 and Figure 6.

When the piston 2 is in the suction or propulsion cycle as shown in Figure 5 and Figure 7, spring 7 is relaxed.

The bidirectional angular movement of the piston 2 acts as a valve for inlet and outlet ports 10, 11 that are located on opposite sides of the hollow cylinder 3. Piston 2 contains two channels 12,13 which cause the inlet port 10 and the outlet port 11 to open and close alternately while the piston 2 moves angularly. At first, the instroke or upstroke of the piston 2 opens the inlet port 10 and closes the outlet port 11, sucking a medical fluid 15 from the inlet port 10 through the first channel 12 into the lower part of the hollow cylinder 3, as shown in Figure 5a and Figure 5b. Then, the outstroke or down stroke of the piston 2 closes the inlet port 10 and opens the outlet port 11, propelling the fluid 15 from said lower part of the pump chamber 3 through the second channel 13 to the outlet port 11, as shown in Figure 7a and Figure 7b. As used in various embodiments, the fluid 15 is a medical fluid that is to be injected into a patient. The medical fluid 15 comprises a contrast medium for injection into a patient. The fluid 15 may also comprise a diluent, such as saline, that is to be injected into a patient.

Said channels 12, 13 have been curve-shaped according to both bidirectional angular and linear movement of the piston 2 in order to ensure a constant opening of the inlet 10 and the outlet 11 during respectively the instroke phase and the outstroke phase of piston 2. This ensures a constant flow of liquid 15 from the inlet port 10 through the piston 2 to the lower part of the cylindrical chamber 3' during the instroke of piston 2 and a constant flow of the liquid 15 from the lower part of the pump chamber 3' to the outlet during the outstroke of the piston 2. Several specifically shaped gaskets or standard O-rings 14 are positioned around the inlet port 10 and the outlet port 11 in order to seal off the existing play between the external diameter of the piston 2 and the internal diameter of the cylindrical chamber 3'. Said gaskets, which comprise specific sealing rib design, are part of the piston 2 or cylinder 3.

The present invention may be adapted for medical use as a parenteral system. The piston 2 and the cylindrical chamber 3' can constitute a disposable. Unlike existing pumps with disposables composed by soft parts such as a flexible membrane or tube as in a peristaltic pump, the disposable piston 2 and cylindrical chamber 3' can be produced by injection molding methods as hard plastic parts and are therefore not influenced by pressure and temperature. As a result, such system allows an accurate release of a specific amount of a drug by a preset angular shift of the rotor 5. A single dose is produced by a 360 degree rotation of said rotor 5. Several doses can be released with such system at fixed intervals of time by simply actuating the rotor.

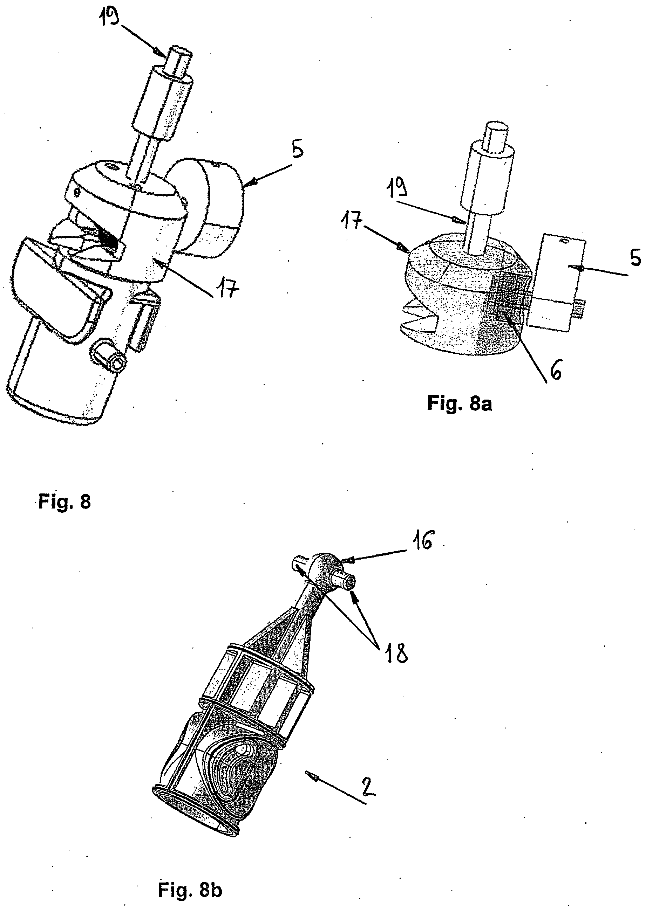

A further example of a pump (not falling within the scope of the present invention) is shown in Figures 8, 8a and 8b which show the upper- end of the piston 2 comprises a ball-and-socket joint 16 which is firmly connected to a piston head 17 through two lugs 18. The rotor 5 bearing the eccentric shaft 6 transmits through piston head 17 a combined bidirectional angular and linear movement to the piston 2, the piston head 17 having a hole into which a shaft 19 is driven in for guidance. Such embodiment avoids abutment which may occur in the example shown in Figures 1 to 7 between the spherical extremity 8 of the shaft 6 and the piston receptacle 9 when the piston 2 is in the suction or propulsion cycle as shown by Figure 5 and Figure 7.

Figures 9 to 15 (which do not fall within the scope of the invention) show a first and a second piston 20, 21 are fixedly positioned opposite to each other inside a hollow cylindrical mobile housing 22 as shown by Figure 9. Said housing 22 is made up of two identical cylindrical parts 23, 23' assembled end-to-end facing each other. A disc 24 shown in Figures 10a, 11, 11 a comprising inlet and outlet ports 10, 11 located preferably laterally at 180 degree from each other and a hole 25 on its underneath part shown in Figure 9a, is mounted midway inside said housing 22 between the two cylindrical parts 23, 23'. Such assembling creates a first and a second chamber 26, 26' shown in Figure 12b, 14b. The disc 24 is angularly movable relative to the housing 22 formed by parts 23, 23'.

A shaft (not shown) is inserted into the hole 25, said shaft being mounted on a rotor 5, as described with respect to Figures 1 to 7, for transmitting to the disc 24 a combined bi-directional linear and angular movement.

Such movement of the disc 24 causes the cylindrical housing 22 to slide back and forth following the axis of the two pistons 20, 21 while closing the inlet and outlet ports 10, 11 so as to ensure on the one hand an alternate sucking of the fluid 15 from the inlet port 10 to respectively the first and second chamber 26, 26' and on the other hand an alternate expelling of the fluid 15 from respectively the first and second chambers 26, 26' to the outlet port 11.

The optimum synchronization of the suction and propulsion phases between the two chambers 26, 26' is achieved by a first and a second T-shaped channel 27, 27' located inside the disc 24 and in its inlet/outlet as shown by Figure 11 a. Channels 27, 27' connect alternately the inlet port 10 to the first and second chambers 26, 26', and the first and the second chamber 26, 26' to the outlet port 11 when said channels 27, 27' overlap alternately the first and the second opening 28, 28' located on the end of both cylindrical parts 23, 23' Figure 10. This particular arrangement allows the volumetric pump to provide a continuous flow. The combined bidirectional linear and angular movement of the piston 2 can be imparted by means of an axle 28 which passes through an upper part 29 rigidly connected with the piston head 17 as shown by Figure 16 and 16a. Said axle 28 can be actuated by at least one rotor 5. The movement of the axle 28 transmits to the piston 2 a movement such as described with respect to Figures 8, 8a and 8b.

Such transmission can be adapted to the invention as shown in Figure 17 and 17a.

The pump 1 can be actuated by two rotors 5, 5' operatively connected to the upper and lower parts of said piston 2 as described with respect to Figures 1 to 7. The first rotor 5 transmits to the piston 2 the movement required by the suction phase while the second rotor 5' transmits to said piston 2 the movement required by the propulsion phase.

All examples of the pump can be adapted so as to dissociate the relative linear movement of the piston with its angular movement. The linear movement can be transmitted by a first rotor and the angular movement can be transmitted by a second rotor. The movement of the piston can be converted from a linear movement to an angular movement at any time of its stroke.

In another variant, the pump 1 can be used as a compressor. A sealed tight tank can be fitted on the outlet port, sucking the air through the inlet 10 into the chamber and propelling the air into the tank by the same mechanism described with respect to Figures 1 to 7. The mechanism of this volumetric pump 1 can also be adapted for an internal combustion engine. Thus, another aspect of the invention is an internal combustion engine comprising a volumetric pump according to the invention, as described herein.

Figure 18 is a block-diagram view of a medical fluid delivery system that includes a volumetric pump. In this embodiment, the medical fluid delivery system is a powered contrast injection system. This system may be used to delivery contrast media for various different types of medical procedures, such as angiographic or computed tomography (CT) procedures.

As shown in Figure 18, the system includes at least two fluid reservoirs. These reservoirs may be bottles, bags, or other forms of containers. At least one fluid reservoir 30 includes contrast media. A fluid reservoir 32 may include a diluent, such as saline. Each fluid reservoir is coupled to a spike/drip chamber assembly 34. Fluid may flow out of the reservoirs and into associated tubing by means of the corresponding spike/drip chamber assembly 34. The pump 41 in the pump assembly 40 draws the fluid out of these reservoirs, as will be described in more detail below. The system selectively controls valves or other selection means 36 to determine which fluid is drawn into the pump 41 (from one of the reservoirs 30 or reservoir 32), and how much fluid is drawn into the pump 41. The system has the capability to individually control each of the valves 36. In one embodiment, pinch valves may be used.

Tubing from each reservoir 30 or 32 is coupled to a connector 38. The connector 38 is coupled to tubing that leads to an input port 39 of the pump 41 in the pump assembly 40. The pump 41 pumps fluid from the input port 39 to an output port 43. The pump 41 may be a volumetric pump as shown in Figure 1, Figure 8 or Figure 9. In one embodiment, the pump 41 is the pump shown in Figure 19a, which is described in more detail below.

The output port 43 of the pump 41 is coupled to tubing that runs through an air/pressure detection unit 42. The unit 42 detects presence of air bubbles of columns, and also detects fluid pressure of the fluid through the associated tubing. The tubing from the pump 41 runs to a patient line that runs to the patient 48. In one embodiment, the patient line runs through a valve, such as a pinch valve or a check valve. In one embodiment, the system is capable of controlling this valve to determine when the pump 41 may inject fluid into the patient.

In some embodiments, various components shown in Figure 18 are single-use components that are used for only a single patient procedure. These components are discarded after each patient procedure. In some embodiments, various components shown in Figure 18 are multi-use components that may be reused across multiple, different patient procedures. In these embodiments, the multi-use components are coupled to certain single-use components (such as the patient line) by way of one or more connectors. Certain components, such as valves (e.g., check valves or pinch valves) may be used in the system in these embodiments. In one embodiment, the reservoir 30, the reservoir 32, the assemblies 34, the valves 36, the tubing leading to the connector 38, and the air/pressure sensor 42 are reusable components, while the tubing from the input port 39, the pump 41, and the tubing from the output port 43

(patient line) are single-use components. In another embodiment, the pump 41 is a reusable component that may be used across multiple patient procedures.

The system of Figure 18 may be used to deliver contrast media and optionally also diluent to the patient. In one embodiment, only one reservoir 30 of contrast media is used. In other embodiments, two or more reservoirs 30 of contrast media may be used. In situations in which no diluent is required for medical injections, the reservoir 32 may be removed from the system.

Figure 19a is a perspective view of a volumetric pump that may be used within the system shown in Figure 18, according to one embodiment. Figure 19a shows an embodiment of the pump 41. The pump 41 is similar to the pump shown in Figure 9.

The pump 41 includes a first hollow cylindrical housing part 52 and a second hollow cylindrical housing part 52'. The cylindrical housing parts 52 and 52' can be assembled end-to-end, facing each other, to form a pump housing. The first and second housing parts 52 and 52' are aligned axially and are coupled by means of housing end units 62 and 62'. A first piston 54 is situated within the first housing part 52, and a second piston 54' is situated within the second housing part 52'. Each piston 54, 54' is fixedly coupled to a stationary component of the system by means of slots 56 and 56'. These slots 56 and 56' are attached to stationary tabs on the system, according to one embodiment, which is shown more clearly in Figure 20.

As shown in the example of Figure 19a, the open slots 56 and 56' are substantially rectangular in cross-sectional shape. In other embodiments, other dimensional configurations may be used for the slots 56 and 56'.

As also shown in the example of Figure 19a, the housing parts 52 and 52' are substantially rectangular in cross-section shape. This can also be seen in Figure 19b. The rectangle-shape parts 52 and 52' can be fitted or assembled together to enclose a disc 64 within. In one embodiment, the disc 64 may be mounted midway inside the pump housing. The resulting three-dimensional structure comprises a semi-open column that may be more easily and effectively used to insert the pump 41 into a housing assembly such as the assembly shown in Figure 20a. In one embodiment, the housing parts 52 and 52' may be square-shaped parts. In one embodiment, a portion of the pump housing, near the assembly of the first and second cylindrical housing parts 52 and 52', is therefore rectangular in shape.

The pump 41 further includes an inlet, or input, port 58 and an outlet, or output, port 60. Fluid flows into a pump chamber of the pump 41 through the input port 58, and flows out of (or expelled from) a pump chamber of the pump 41 from the output port 60. An arrow is included on a component adjacent to the output port 60 to indicate the direction in which the pump 41 is to be inserted, or installed, in a housing assembly.

Figure 19b is a perspective view of individual components of the pump shown in Figure 19a. This figure shows how the housing end units 62 and 62' may be coupled together and around the ports 58, 60. The ports 58 and 60 are coupled to a rotatable disc 64, as shown in the figure. The disc 64 includes two fluid ports, which comprise holes in one embodiment. The disc 64 rotates back and forth in bidirectional angular motion to provide fluid flow into or out of the ports 58, 60 by ways of these fluid ports in the disc 64. During a first cycle, fluid is drawn into the input port 58 and into a first cylinder within the first housing part 52. Fluid is also expelled from a second cylinder within the second housing part 52' to the output port 60. During a second cycle, fluid is drawn into the input port 58 and into the second cylinder, while fluid is expelled form the first cylinder to the output port 60. In one embodiment, each of the first and second cylinders is capable of holding 5 ml of fluid. An arrow is included on a component adjacent to the output port 60 to indicate the direction in which the pump 41 is to be inserted, or installed, in a housing assembly. In one embodiment, the pump 41 is arranged to be animated by a preferably combined bidirectional linear and angular movement to cause sliding between the pump housing and the pistons 54 and 54' along the axis of said pistons while closing the inlet and outlets ports in a synchronized fashion to provide a substantially continuous delivery of medical fluid to a patient.

Rather than being actuated by a shaft mounted on a rotor, such as described previously with respect to Figure 9, the disc 64 of the pump 41 shown in Figures 19a and 19b is driven by a drive member that becomes operatively coupled to the C- or arc-shaped receptacle of the disc 64, as better seen in Figure 19b. In one embodiment, this receptacle may comprise a socket into which an end of the drive member, such as a ball-shaped end, may be coupled and engaged. The drive member (not shown Figure 19a or 19b, but is shown in Figure 20c as component 102) is capable of actuating the disc 64 in angular, or rotation, motion during operation. Linear movement of the drive member causes rotational, or angular, movement of the disc 64. During each cycle, the disc 64 is capable of rotating in the opposite direction than its movement during the previous cycle.

In one embodiment, the pistons 54 and 54', along with the disc 64, are made of a Ticona material. In one embodiment, the housing parts 52, 52' and end units 62, 62' are made of a polycarbonate material. In one embodiment, the housing parts 52, 52' and end units 62, 62' are made of ABS (acrylonitrile butadiene styrene).

Figure 20a is a perspective view of a housing assembly that contains the volumetric pump of Figure 19a, according to one embodiment. The assembly includes frames 70, rods 76, housing supports 72, piston holders 73, tabs 77, a motor holder 71, a motor shaft 75, a motor 74, and the pump 41. The motor 74 drives the assembly for bi-directional, linear motion along the rods 76. In the embodiment shown in Figure 20a, the pump 41 is coupled to the tabs 77 of the assembly. In one embodiment, the tabs 77 are stationary and protrude from the assembly. The pump 41 is loaded into the assembly, such that the slots 56 and 56' are fitted around the tabs 77. The tabs 77 keep the pump 41 in place. In this embodiment, the pump 41 and tabs 77 are held stationary while the motor moves the rest of the housing assembly in a back-and-forth motion along the rods 76. This movement of the assembly causes fluid to be drawn in and expelled out of the chambers (reciprocally) of the pump 41, as described above.

In one embodiment, the actuator of the motor 74 is a belt-gear driven elliptical cam system using a 120 Watt Maxon motor. The system is capable of providing various flow rates of fluid injection into a patient, as well as various fluid pressures. In one embodiment, the pump 41 supports flow rates between 1-18 ml/s, and pressure of up to 450 psi. In other embodiments, various different flow rates and fluid pressures may be provided, such as for angiographic procedures, where higher pressures may be required. In one embodiment, flow rates 0.8-40 ml/s and fluid pressures 200-1200 psi may be provided.

Figures 20b and 20c show views of portions of the assembly of Figure 20a, according to one embodiment. Figure 20b shows the rods 76 along which the assembly slides in a linear, bi-directional manner. Figure 20b also shows an elliptical cam 78, a pin 79, and a drive link 100. The motor 74 drives the elliptical cam 78, and the pin 79 is a stationary pin 79 in one embodiment. The movement of the elliptical cam 78 causes movement of the drive link 100. Movement of the drive link 100 causes bi-directional, linear movement of the assembly along the rods 76.

Figure 20c shows another perspective view of a portion of the assembly. As shown in this figure, movement of the elliptical cam 78 also causes bi-directional, linear movement of a valve drive member 102. In one embodiment, movement of the drive member 102 is perpendicular to movement of the housing assembly along the rods 76. The driver member 102 is coupled to a socket of the disc 64 (in the pump 41), which can be seen from Figure 19b. Linear movement of the driver member 102 causes rotational movement of the disc 64. The combination of movement of the housing assembly along the rods 76 with the rotational movement of the disc 64 causes fluid to be reciprocally drawn into and expelled from the chambers of the pump 41, according to one embodiment. In one embodiment, these movements reduce pulsatile flow of fluid expelled from the pump 41.

Figure 21 a is a view of a portion of the system shown in Figure 18, according to one embodiment. The portion that is shown includes a fluid reservoir 80 (which can be either the reservoir 30 or 32 in one embodiment), a spike 82, an air vent 85, a drip chamber 84, tubing 86, and the pump assembly 40. The spike 82, the air vent 85, and the drip chamber 84 are part of the spike/drip chamber assembly 34 discussed previously, according to one embodiment. During operation, the pump assembly 40 and pump 41 continuously draw in medical fluid from the reservoir 80 at one or more specified flow rates. In one embodiment, the pump assembly 40 can draw in fluid at a rate of 18ml/s.

In the embodiment shown in Figure 21a, the tubing 86 that leads to the pump assembly 40 has a length of twenty-one inches (about 50cm), and outer diameter of 0.25 inches (about 6.4mm), and an inner diameter of 0.160 inches (about 4.1 mm). In other embodiments, various other dimensions for tubing 86 may be used. Although not shown in Figure 21a, the tubing 86 also may pass through a valve, such as the valve 36 shown in Figure 18. In one embodiment, the air vent 85 has a five micron air filter.

Figure 21b is a cross-sectional view of a portion of the spike assembly that is shown in Figure 21a along line A-A, according to one embodiment. This cross-sectional view shows a semicircular spike inlet 87 (for fluid flow) and a circular air vent 88. The semicircular spike inlet 87 has a diameter L1, and the diameter of the cross-section of spike assembly along A-A is L2. In one embodiment, L1 equals 0.125 inches (about 3.2mm) and L2 equals 0.200 inches (about 5.1 mm). In other embodiments, L1 and L2 may have different dimensions.

Figure 22 is a side sectional view of a portion of a piston that is included within the pump shown in Figure 19a, according to one embodiment. The portion 90 that is shown in Figure 22 is a portion of either the piston 54 or 54' shown in Figure 19a in this embodiment. The portion 90 includes a piston outer surface 92, which comes into direct contact with the fluid to be pressurized by the pump. The portion 90 further includes two sealing members 94 and 96 that extend around the periphery, or circumference, of the piston. The sealing members 94 and 96 help provide improved sealing functionality. In one embodiment, these members 94 and 96 are coupled with the inner wall of the chamber (with the part 52 or 52' shown in Figure 19a) to provide a lower coefficient of friction than if a greater amount of the portion 90 were to be coupled with the inner wall. In one embodiment, the sealing members 94 and 96 may comprise a material similar to the material used in the remainder of the portion 90 of the piston. In one embodiment, additional sealing members extending around the periphery, or circumference, of the piston may be used, in addition to member 94 and 96, to further improve the sealing functionality.

In one embodiment, the sealing members 94 and 96 comprise gaskets that may be made of a rubber material, such as EDPM (ethylene-diene-propylene-monomer). In other embodiments, various other material types may be used.

In one embodiment, the sealing members 94 and 96 comprise over-molded components (over the piston). In one embodiment, the sealing members 94 and 96 may be made of a plastic material that is over-molded on the piston. In one embodiment, the sealing members 94 and 96 may be made of a material similar to the material used in the remainder of the portion 90 of the piston. In one embodiment, over-molded members 94 and 96 may potentially help improve sealing functionality at higher injection pressures.

## Claims

1. A fluid delivery system comprising:
a volumetric pump (41); and
one or more reservoirs (30) coupled to the pump (41),
wherein the pump (41) comprises:
at least one first piston (54) inside a first hollow cylindrical part (52);
at least one second piston (54') positioned opposite to the first piston inside a second hollow cylindrical part (52'), both cylindrical parts (52, 52') being assembled end-to-end facing each other to form a housing;
at least one inlet port (58) through which a medical fluid can flow into at least one pump chamber;
at least one outlet port (60) through which the medical fluid can be expelled; and
a drive link (100) providing a bidirectional linear movement to cause sliding between the housing (22) and the pistons (54, 54') along the axis of the pistons,
**characterised in that**
at least one of the one or more reservoirs includes a contrast media;
the fluid delivered comprises a medical fluid including the contrast media; and
the pump comprises a disc (64) mounted midway inside said housing, the disc (64) comprising the inlet and outlet ports (58, 60), the disc (64) including a receptacle to receive a drive member (102), wherein linear movement of the drive member (102) causes angular movement of the disc (64) to close the inlet and outlets ports (58, 60) in a synchronized fashion to provide a substantially continuous delivery of the medical fluid to a patient.

2. The fluid delivery system according to claim 1, wherein the receptacle comprises a socket to receive a ball-shaped end of the drive member (102).

3. The fluid delivery system according to claim 2 , further comprising a spike/drip chamber assembly (34) coupled to each of the one or more reservoirs (30) of contrast media.

4. The fluid delivery system according to claim 3, further comprising a valve (36) located between the one or more reservoirs (30) of contrast media (30) and the pump (41).

5. The fluid delivery system according to claim 1, further comprising an air/pressure sensor (42) coupled to the pump (41).

6. The fluid delivery system according to claim 1, wherein the system is a powered contrast injection system.

7. The fluid delivery system according to claim 1, further comprising a reservoir of diluents (32) coupled to the pump (41).

8. The fluid delivery system according to claim 1, wherein a portion of said housing, near the assembly of the first and second cylindrical parts (52, 52') is rectangular in shape.

9. The fluid delivery system according to claim 1, wherein movement of the drive member (102) is perpendicular to the movement of the drive link.

## Patentansprüche

1. Fluidzuführungssystem, das Folgendes umfasst:
eine volumetrische Pumpe (41), und
ein oder mehrere mit der Pumpe (41) gekoppelte Reservoire (30),
wobei die Pumpe (41) Folgendes umfasst:
wenigstens einen ersten Kolben (54) innerhalb eines ersten hohlzylindrischen Teils (52);
wenigstens einen zweiten Kolben (54'), der gegenüber dem ersten Kolben innerhalb eines zweiten hohlzylindrischen Teils (52') positioniert ist, wobei beide zylindrische Teile (52, 52') Ende an Ende einander zugewandt zusammengefügt sind, um ein Gehäuse zu bilden;
wenigstens einen Einlassanschluss (58), durch den ein medizinisches Fluid in wenigstens eine Pumpenkammer fließen kann;
wenigstens einen Auslassanschluss (60), durch den das medizinische Fluid ausgestoßen werden kann; und
eine Antriebsverbindung (100), die eine bidirektionale Linearbewegung erzeugt, um ein Gleiten zwischen dem Gehäuse (22) und den Kolben (54, 54') entlang der Achse der Kolben zu bewirken,
**dadurch gekennzeichnet, dass**
wenigstens eines der ein oder mehreren Reservoire ein Kontrastmedium beinhaltet;
das zugeführte Fluid ein medizinisches Fluid einschließlich des Kontrastmediums umfasst; und
die Pumpe eine Scheibe (64) umfasst, die in der Mitte innerhalb des genannten Gehäuses montiert ist, wobei die genannte Scheibe (64) die Ein- und Auslassanschlüsse (58, 60) umfasst, wobei die Scheibe (64) eine Aufnahme zum Aufnehmen eines Antriebselements (102) beinhaltet, wobei eine Linearbewegung des Antriebselements (102) eine Winkelbewegung der Scheibe (64) bewirkt, um die Ein- und Auslassanschlüsse (58, 60) auf synchronisierte Weise zu schließen, um eine im Wesentlichen kontinuierliche Zuführung des medizinischen Fluids zu einem Patienten zu ermöglichen.

2. Fluidzuführungssystem nach Anspruch 1, wobei die Aufnahme eine Fassung zum Aufnehmen eines kugelförmigen Endes des Antriebselements (102) umfasst.

3. Fluidzuführungssystem nach Anspruch 2, das ferner eine Spike/Tropfkammer-Baugruppe (34) umfasst, die mit jedem der ein oder mehreren Kontrastmediumreservoirs (30) gekoppelt ist.

4. Fluidzuführungssystem nach Anspruch 3, das ferner ein Ventil (36) umfasst, das sich zwischen den ein oder mehreren Kontrastmediumreservoirs (30) und der Pumpe (41) befindet.

5. Fluidzuführungssystem nach Anspruch 1, das ferner einen mit der Pumpe (41) gekoppelten Luft/Druck-Sensor (42) umfasst.

6. Fluidzuführungssystem nach Anspruch 1, wobei das System ein kraftgetriebenes Kontrastmittelinjektionssystem ist.

7. Fluidzuführungssystem nach Anspruch 1, das ferner ein mit der Pumpe (41) gekoppeltes Reservoir von Verdünnungsmitteln (32) umfasst.

8. Fluidzuführungssystem nach Anspruch 1, wobei ein Teil des genannten Gehäuses in der Nähe der Baugruppe des ersten und zweiten zylindrischen Teils (52, 52') rechteckig geformt ist.

9. Fluidzuführungssystem nach Anspruch 1, wobei die Bewegung des Antriebselements (102) lotrecht zur Bewegung der Antriebsverbindung ist.

## Revendications

1. Système d'administration de fluide comprenant :
une pompe volumétrique (41) ; et
un ou plusieurs réservoirs (30) couplés à la pompe (41),
dans lequel la pompe (41) comprend :
au moins un premier piston (54) à l'intérieur d'une première pièce cylindrique creuse (52) ;
au moins un second piston (54') positionné opposé au premier piston à l'intérieur d'une seconde pièce cylindrique creuse (52'), les deux pièces cylindriques (52, 52') étant assemblées bout-à-bout l'une en face de l'autre pour former un logement ;
au moins un orifice d'entrée (58) par lequel un fluide médical peut s'écouler jusque dans au moins une chambre de pompe ;
au moins un orifice de sortie (60) par lequel le fluide médical peut être expulsé ; et
une liaison d'entraînement (100) fournissant un mouvement linéaire bidirectionnel pour entraîner un glissement entre le logement (22) et les pistons (54, 54') le long de l'axe des pistons,
**caractérisé en ce que**
au moins l'un des un ou plusieurs réservoirs comportent un milieu de contraste ;
le fluide administré comprend un fluide médical comportant le milieu de contraste ; et
la pompe comprend un disque (64) monté à mi-chemin à l'intérieur dudit logement, le disque (64) comprenant les ports d'entrée et de sortie (58, 60), le disque (64) comportant un réceptacle pour recevoir un élément d'entraînement (102), dans lequel un mouvement linéaire de l'élément d'entraînement (102) entraîne un mouvement angulaire du disque (64) pour fermer les ports d'entrée et de sortie (58, 60) de manière synchronisée pour assurer une administration sensiblement continue du fluide médical à un patient.

2. Système d'administration de fluide selon la revendication 1, dans lequel le réceptacle comprend un socle pour recevoir une extrémité en forme de bille de l'élément d'entraînement (102).

3. Système d'administration de fluide selon la revendication 2, comprenant en outre un ensemble de perforateur/chambre compte-gouttes (34) couplé à chacun des un ou plusieurs réservoirs (30) de milieu de contraste.

4. Système d'administration de fluide selon la revendication 3, comprenant en outre un clapet (36) situé entre les un ou plusieurs réservoirs (30) de milieu de contraste (30) et la pompe (41).

5. Système d'administration de fluide selon la revendication 1, comprenant en outre un capteur d'air/pression (42) couplé à la pompe (41).

6. Système d'administration de fluide selon la revendication 1, le système étant un système à injection de contraste motorisé.

7. Système d'administration de fluide selon la revendication 2, comprenant en outre un réservoir de diluants (32) couplé à la pompe (41).

8. Système d'administration de fluide selon la revendication 1, dans lequel une partie dudit logement, près de l'ensemble des première et seconde pièces cylindriques (52, 52') est de forme rectangulaire.

9. Système d'administration de fluide selon la revendication 1, dans lequel le mouvement de l'élément d'entraînement (102) est perpendiculaire au mouvement de la liaison d'entraînement.
